(19) Europäisches Patentamt
European Patent Office
Office européen des brevets

(11) Veröffentlichungsnummer: **0 177 907**
**B1**

(12) # EUROPÄISCHE PATENTSCHRIFT

(45) Veröffentlichungstag der Patentschrift: **01.06.88**

(51) Int. Cl.⁴: **C 07 D 213/70**, A 61 K 31/44

(21) Anmeldenummer: **85112599.7**

(22) Anmeldetag: **04.10.85**

(54) **Neue 2-Pyridin-thiol-Derivate, Verfahren zu ihrer Herstellung und diese enthaltende pharmazeutische Präparate.**

(30) Priorität: **05.10.84 HU 377584**

(43) Veröffentlichungstag der Anmeldung: **16.04.86 Patentblatt 86/16**

(45) Bekanntmachung des Hinweises auf die Patenterteilung: **01.06.88 Patentblatt 88/22**

(84) Benannte Vertragsstaaten: **AT BE CH DE FR GB IT LI NL SE**

(56) Entgegenhaltungen:
**AT - B - 238 200**
**US - A - 4 374 992**

**CHEMICAL ABSTRACTS, Band 96, Nr. 5, 1. Februar 1982, Columbus, Ohio, USA YOSHITOMI "Pyridine derivatives" Seite 652, Spalte 2, Zusammenfassung-Nr. 35096v**

(73) Patentinhaber: **Richter Gedeon Vegyészeti Gyár R.T., Gyömröi ut 19-21, H-1475 Budapest X (HU)**

(72) Erfinder: **Ezer, Elemér, Lupény u. 6-8, H-1026 Budapest (HU)**
Erfinder: **Harsányi, Kálmán, Dr., Fodor u. 12, H-1126 Budapest (HU)**
Erfinder: **Vikár geb. Pethö, Hajnalka, Süveg u. 16, H-1112 Budapest (HU)**
Erfinder: **Matuz, Judit, Özgida u. 32, H-1025 Budapest (HU)**
Erfinder: **Szporny, Lászlo, Dr., Szabolcska u. 7, H-1114 Budapest (HU)**
Erfinder: **Cholnoky, Eszter, Dr., Bartok Béla u. 4, H-1111 Budapest (HU)**
Erfinder: **Kuthi, Csaba, Bürök u. 24, H-1124 Budapest (HU)**
Erfinder: **Trischler, Ferenc, Dr., Uttörö u. 16, H-1171 Budapest (HU)**
Erfinder: **Hegedüs, Béla, Dr., Bartok Béla u. 82, H-1113 Budapest (HU)**
Erfinder: **Kápolnás geb. Pap, Márta, 525 tér 33, H-1173 Budapest (HU)**
Erfinder: **Kállay geb. Sohonyai, Anna, Csertö u. 18-20, H-1144 Budapest (HU)**

(74) Vertreter: **Lehn, Werner, Dipl.-Ing. et al, Hoffmann, Eitle & Partner Patentanwälte Arabellastrasse 4, D-8000 München 81 (DE)**

**EP 0 177 907 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue 2-Pyridin-thiol-Derivate, Verfahren zu ihrer Herstellung und diese enthaltende pharmazeutische Präparate. Gegenstand der Erfindung sind also neue 2-Pyridin-thiol-Derivate der allgemeinen Formel I

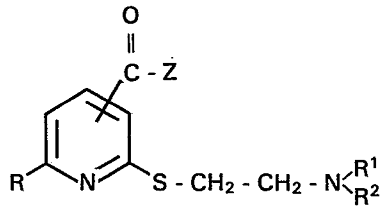
(I)

worin

R für ein Wasserstoffatom oder eine Alkylgruppe mit 1-4 Kohlenstoffatomen steht,

Z gegebenenfalls für eine durch ein oder mehrere Halogenatome und/oder für eine durch eine Alkylgruppe mit 1-4 Kohlenstoffatomen substituierte Phenylgruppe steht,

$R^1$ und $R^2$ unabhängig voneinander die Bedeutung eines Wasserstoffatoms, einer Alkylgruppe mit 1-4 Kohlenstoffatomen, einer Alkyl-phenylgruppe mit 7-11 Kohlenstoffatomen oder einer Gruppe der allgemeinen Formel $R-\overset{\overset{O}{\|}}{C}-$ besitzen, wobei die Bedeutung von R die obige ist,

und der Substituent $-\overset{\overset{O}{\|}}{C}-Z$ bindet sich an die 3- oder 4-Stelle des Pyridinringes,

mit der Festlegung, dass falls sowohl $R^1$ als auch $R^2$ die Bedeutung einer Methylgruppe besitzen, und die Bedeutung von Z eine 4-Chlor-phenylgruppe ist, R nicht Wasserstoffatom bedeutet.

Gemäss der Erfindung geht die Herstellung der 2-Pyridin-thiol-Derivate der allgemeinen Formel I und ihrer Säureadditionssalze wie folgt vor sich:

a) ein 2-Halogen-pyridin-Derivat der allgemeinen Formel II

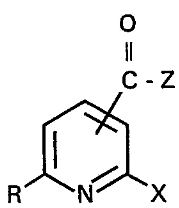
(II)

worin die Bedeutung von R und Z die obige, die von X Halogenatom ist,

wird mit einer Thiolverbindung der allgemeinen Formell III

$$HS-CH_2-CH_2-N{<}^{R^1}_{R^2} \qquad (III)$$

worin die Bedeutung von $R^1$ und $R^2$ wie oben ist,

oder deren Säureadditionssalz umgesetzt; oder

b) ein Pyridin-2-thion-Derivat der allgemeinen Formel IV

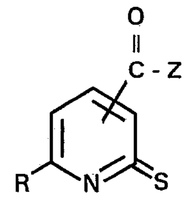
(IV)

worin die Bedeutung von R und Z die obige ist,

wird mit einem 2-Halogen-äthyl-amin-Derivat der allgemeinen Formel V

$$X-CH_2-CH_2-N{<}^{R^1}_{R^2} \qquad (V)$$

worin die Bedeutung von $R^1$ und $R^2$ die obige, die von X Halogenatom ist,

oder dessen Säureadditionssalz umgesetzt; oder

c) ein reaktionsfähiges Derivat einer 2-Hydroxy-äthyl-thio-pyridin-Verbindung der allgemeinen Formel VI

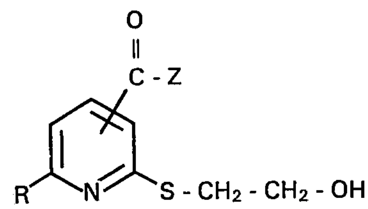
(VI)

worin die Bedeutung von R und Z die obige ist,

vorzugsweise ein entsprechendes Halogenid oder dessen Säureadditionssalz, wird mit einem Amin der allgemeinen Formel VII

$$HN{<}^{R^1}_{R^2} \qquad (VII)$$

worin $R^1$ und $R^2$ die obige Bedeutung besitzen,

umgesetzt; oder

d) zur Herstellung von Verbindungen der allgemeinen Formel I, worin $R^1$ und $R^2$ gleichzeitig für ein Wasserstoffatom stehen, Z und R die vorherige Bedeutung haben, wird ein 2-Halogen-äthyl-thio-pyridin-Derivat der allgemeinen Formel VIII

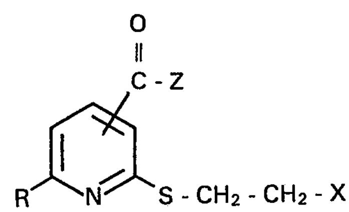
(VIII)

worin die Bedeutung von R, Z und X die obige ist,

mit Phthalimid-kalium umgesetzt, dann wird die Phthaloyl-Schutzgruppe auf an sich bekannte Weise entfernt, und

gewünschtenfalls wird der $R^1$- oder $R^2$-Substituent einer im Verfahren a), b), c) oder d) hergestellten Verbindung der allgemeinen Formel I zu einem anderen $R^1$- bzw. $R^2$-Substituenten konvertiert, der unter eine oben gegebene Definition fällt, und/oder

gewünschtenfalls wird eine erhaltene Verbindung der allgemeinen Formel I zu ihrem Säureadditionssalz konvertiert oder aus ihrem Säureadditionssalz freigesetzt.

In den obengenannten allgemeinen Formeln können R, $R^1$ und $R^2$ als Alkylgruppen mit 1-4 Kohlenstoffatomen die Bedeutung von Gruppen mit linearen oder verzweigten Kohlenstoffketten,wie Methyl-, Äthyl-, n-Propyl-, i-Propyl-, n-Butyl-, sec-Butyl- und tert-Butylgruppen besitzen.

In der Bedeutung von $R^1$ und $R^2$ kann die Alkylphenylgruppe mit 7-11 Kohlenstoffatomen im Alkylteil die obigen Alkylgruppen mit linearen oder verzweigten Kohlenstoffketten enthalten, und ist vorzugsweise eine Benzylgruppe.

In der Bedeutung von Z ist die Phenylgruppe vorzugsweise nicht substituiert oder trägt einen oder zwei Substituenten. Als Substituenten sind das Chloratom und die Methylgruppe besonders vorteilhaft.

Die Verbindungen der allgemeinen Formel I sind therapeutisch aktiv, insbesondere zeigen sie eine sehr wertvolle gastrocytoprotektive Wirkung. Gegenstand der Erfindung sind demnach auch pharmazeutische Präparate, die als Wirkstoff eine Verbindung der allgemeinen Formel I oder deren therapeutisch akzeptierbare Säureadditionssalze enthalten.

Mit Verbindungen analoger Struktur befasst sich die unter der Nr.56-100 765 offengelegte japanische Patentanmeldung Nr.55-2920, deren weit gefasster Begriff der allgemeinen Formel I auch einen Teil der erfindungsgemässe Verbindungen beinhaltet. Diese Anmeldung beschreibt jedoch als Beispiel nur eine einzige Verbindung, in der sich die Amino-äthyl-Seitenkette über eine Thiogruppe an den Pyridinring bindet, namentlich das in Beispiel 2 vorkommende 2-(2-Dimethyl-amino-äthyl-thio)-3-(4-chlor-benzoyl)-pyridin. Alle weiteren, auch konkret beschriebenen Verbindungen sind Alkyl- und Dialkyl-amino-alkoxy- oder (zu einem geringen Teil) N',-N'-Dialkyl-amino-N-alkyl-amino-pyridin-Derivate. Gleichzeitig erwähnt die genannte Patentanmeldung lediglich die Anti-Apomorphin-, Muskelrelaxans-, beruhigende und den Hirnkreislauf beeinflussende Wirkung, ohne statistische pharmazeutische Angaben zu machen. In der Anmeldung ist nicht der geringste Hinweis darauf zu finden, dass die Verbindungen Anti-Geschwür-Wirkung, so gastrocytoprotektive Wirkung, zeigen.

Von den Pyridinverbindungen tauchten früher einzelne Pyridyl-essigsäure-thioamid-Derivate bei der Therapie von Magen- und Darmgeschwüren auf. Solche sind das die Magensäuresekretion hemmende Thiquinamid · HCl (Wy-24 081), das antigastrine α-Phenyl-2-pyridin-äthan-thioamid (SC-15 395) und das α-Methoxy-N-methyl-2-pyridin-äthan-thioamid (SKF-59 377). Über die 2-Pyridyl-karbamide als magensäuresekretionshemmende Stoffe erschien in letzter Zeit eine Veröffentlichung [J. Med. Chem. 26, 538 (1983)]. All diese Verbindungen weichen in ihrer Struktur stark von den neuen erfindungsgemässen Verbindungen ab.

Aus der US-A-4 374 992 sind am Pyridinring durch eine Phenylalkylthiogruppe substituierte Verbindungen und deren quaternäre Salze bekannt, die eine Antiulcus oder antisekretorische Aktivität haben.

In der Geschwürtherapie gelangten in der letzten Zeit neben den als üblich zu betrachtenden säuremindernden Mitteln die sogenannten cytoprotektiven Stoffe in den Vordergrund. Den Begriff der Cytoprotektion führte in der Geschwürforschung im Zusammenhang mit den Prostaglandinen A. Robert [Gastroenterology, 77:761-167 (1979)] ein, der Wirkungsmechanismus der Cytoprotektion ist jedoch bis heute nicht geklärt. Nach Robert spielen bei der cytoprotektiven Wirkung die Prostaglandine die Hauptrolle, während in jüngster Zeit bewiesen wurde, dass auch den sogenannten Sulfhydrylen eine wichtige Rolle zukommt [Szabó und Mitarbeiter: Science, 214, 200-2 (1981)].

Überraschenderweise wurde gefunden, dass die in keiner Weise mit den Prostaglandinen verwandten neuen 2-Pyridin-thio-Derivate der allgemeinen Formel I gegenüber experimentell ausgelösten Magenschäden etwa die gleiche Wirksamkeit aufweisen wie diese. In niedriger Dosis zeigen sie cytoprotektive Wirkung, während sie in im Verhältnis dazu 100- bis 500facher Dosis die Magensäuresekretion senken.

Die pharmakologische Wirkung der Verbindungen wurde in den folgenden Tests untersucht.

*Untersuchung von durch saures Äthanol ausgelösten Magenschäden (cytoprotektive Wirkung)*

Für die Untersuchungen wurden weibliche RG-Wistar-Ratten mit einem Gewicht von 120-150 g verwendet. Die Tiere liess man 24 Stunden lang hungern, während sie Wasser bekamen. Zur Irritierung des Magens wurde ein Gemisch aus 1 ml konzentrierter Salzsäure und 50 ml absolutem Äthanol in einer Dosis von 0,5 ml/g Körpergewicht oral angewendet. Die zu untersuchenden Verbindungen wurden ebenfalls oral, 30 Minuten vor der Säure-Äthanol-Behandlung verabreicht, die Tiere wurden eine Stunde später mittels Äthernarkose eingeschläfert. Der Magen wurde entfernt, dann geöffnet. Nach dem Reinigen wurde das Nassgewicht des Magens bestimmt, danach wurde — im Interesse der Bestimmung des Magenödems — der Unterschied zwischen dem erhaltenen Nassgewicht und dem Nassgewicht des Magens der unbehandelten (Kontroll-) Tiere berechnet. Danach wurden die Magen getrocknet und die Magenschäden visuell betrachtet. Zur Charakterisierung des Ausmasses der Schäden wurde die in mm angegebene durchschnittliche Länge der Magenschädigung verwendet. Die statistische Auswertung der Ergebnisse erfolgte mit der Student-Probe.

Die Ergebnisse des obigen Versuchs im Zusammenhang mit einer als besonders wirksam befundenen, bekannten Verbindung (Cimetidin) und die $ED_{50}$-Werte einiger erfindungsgemässer Verbindungen werden in Tabelle 1 bzw. 2 angegeben.

Untersuchte Verbindungen:

A = 2-([2-Amino-äthyl]-thio)-3-benzoyl-pyridin HCL

B = 3-Benzoyl-2-{(2-[N,N-diacetyl-amino]-äthyl)-thio}-pyridin

C = 2-([2-Amino-äthyl]-thio)-3-[p-chlor-benzoyl]-pyridin · HCl

D = 2-([2-Amino-äthyl]-thio)-3-[2,5-dimethyl-benzoyl]-pyridin · HCl

E = 2-([2-Amino-äthyl]-thio)-4-benzoyl-6-propyl-pyridin · 2 HCl

TABELLE 1

Untersuchung von durch saures Äthanol ausgelösten Magenschäden

| Vorbehandlung | N | Dosis (mg/kg) p.o. | Ödem (mg) | Hemmung (%) | Hämorrhagische Schädigung (mm) | Hemmung (%) |
|---|---|---|---|---|---|---|
| Säure-Äthanol-Kontrolle | 25 | — | 379±34 | — | 85±15 | — |
| A | 8 | 0,05 | 307±42 | 19 | 48±19 | 45 |
| A | 10 | 0,1 | 209±51 | 45* | 38±18 | 56* |
| A | 12 | 1,0 | 72±28 | 82** | 5±1,5 | 96** |
| A | 12 | 10,0 | 22±12 | 95** | 6±3 | 94** |
| Cimetidin | 8 | 25 | 441±82 | — | 82±21 | 2 |
| Cimetidin | 8 | 100 | 301±42 | 21 | 42±13 | 46 |

* $p < 0,05$ im Vergleich zur Säure-Äthanol-Kontrollgruppe
** $p < 0,01$

TABELLE 2

$ED_{50}$-Wert einiger Verbindungen im Säure-Äthanol-Test

| Untersuchte Verbindung | Magenödem-Hemmung $ED_{50}$ (mg/kg p.o.) | Hämorrhagische Schädigung - Hemmung $ED_{50}$ (mg/kg p.o.) |
|---|---|---|
| A | 0,2 | 0,1 |
| B | 1,0 | 2,0 |
| C | 10,0 | 15,0 |
| D | 10,0 | 10,0 |
| E | 20,0 | 25,0 |

*In Shay-Ratten hervorgerufene magensäuresekretionssenkende Wirkung*
[Gastroenterology, 5, 43-46 (1945)]

Weibliche H-Wistar-Ratten mit einem Gewicht von 120-150 g liess man 24 Stunden lang hungern, während die Tiere Wasser bekamen. Unter schwacher Äthernarkose wurde die Pylorus der Tiere abgebunden. Die zu untersuchenden Verbindungen wurden während des Eingriffs teils oral, teils intraperitoneal verabreicht. Die Ratten wurden nach 4 Stunden mittels Äthernarkose eingeschläfert, nach Herausnehmen des Magens wurden Volumen und pH des Mageninhaltes gemessen und die Säuremenge durch Titrieren bestimmt.

Die Versuchsergebnisse sind in Tabelle 3 zusammengefasst.

TABELLE 3

In Shay-Ratten hervorgerufene magensäuresekretionshemmende Wirkung
(orale und intraperitoneale Behandlung)

| Behandlung | N | Dosis (mg/kg) | Ausscheidung HCl/4 h (µmol/100 g Körpergewicht) | Ausscheidung HCl-Hemmung (%) |
|---|---|---|---|---|
| Kontrolle | 10 | — | 564±42 | — |
| A | 5 | 5 p.o. | 357±35 | 37 |
| A | 15 | 10 p.o. | 350±38 | 38 |
| A | 15 | 20 p.o. | 372±40 | 34 |
| A | 5 | 40 p.o. | 124±27 | 70* |
| A | 5 | 6 i.p. | 505±29 | 11 |
| A | 10 | 12 i.p. | 200±30 | 65* |
| A | 5 | 25 i.p. | 0 | 100 |

* $p < 0,001$ verglichen mit der Kontrollgruppe $ED_{50}$: 50 mg/kg

*Hemmung von durch Indomethazin ausgelösten antralen und intestinalen Geschwüren*

Weibliche RG-Wistar-Ratten mit einem Gewicht von 120-150 g liess man 24 Stunden lang hungern, während sie Wasser bekamen. Danach liess man die Tiere eine Stunde lang an Nahrung herankommen, dann wurden sie 30 Minuten nach der Behandlung mit der zu untersuchenden Verbindung oral mit Indomethazin in einer Dosis von 15 mg/kg behandelt. Die Ratten wurden 24 Stunden nach der Indomethazin-Behandlung mittels Äthernarkose eingeschläfert. Nach Entfernen des Magens und des gesamten Dünndarmes wurde der Magen geöffnet, dann die Summe der Geschwürgebiete bestimmt (Geschwür-Index, $mm^2$).

Für die Bewertung der Dünndarmgeschwüre wurde die sogenannte Aufblasmethode [Ezer E., Szporny L. und Mitarbeiter, J. Pharm. Pharmac. 27,866 bis 867 (1975)] verwendet. Die Zerreissfestigkeit des Dünndarmes (ZFDD), angegeben in mm Hg, sinkt gut messbar mit fortschreitender Geschwürbildung. Die statistische Auswertung der Ergebnisse wurde mit der Student-Probe durchgeführt. Die erhaltenen Ergebnisse sind in Tabelle 4 zusammengefasst.

TABELLE 4

Hemmung von durch Indomethazin ausgelösten Geschwüren

| Behandlung | N | Dosis (mg/kg p.o.) | Intestinales Geschwür ZFDD 24 h nach Indomethazin-Behandlung (mm Hg) | Antrales Geschwür Geschwür-Index ($mm^2$/Magen) | Antrales Geschwür Ratten ohne Geschwür |
|---|---|---|---|---|---|
| Indomethazin-Kontrolle | 50 | 15 + Trägermaterial | 147±11 | 14,8 | 15 |
| Indomethazin + A | 10 | 15 + 5 | 162±14 | 18,9 | 3 |
| Indomethazin + A | 20 | 15 + 25 | 198±9* | 4,0* | 12* |
| Indomethazin. + Pirenzipin | 20 | 15 + 25 | 152±8 | 12,0 | 5 |
| Indomethazin + Cimetidin | 10 | 15 + 50 | 161±8 | 14,7 | 2 |

* $p < 0,01$ im Vergleich zur mit Indomethazin behandelten Kontrollgruppe

*Hemmung von durch Aspirin hervorgerufenen Magengeschwüren*

Weibliche H-Wistar-Ratten mit einem Gewicht von 120-150 g liess man 24 Stundenlang hungern, während sie Wasser bekamen. Am Drüsenteil des Magens wurden durch orale Gabe von mit Tween 80 angefertigter Aspirin-Suspension in einer Dosis von 100 mg/kg Magengeschwüre erzeugt. Der zu untersuchende Stoff wurde den Tieren gleichzeitig mit dem Aspirin, ebenfalls oral verabreicht. Nach 4 Stunden wurden die Tiere eingeschläfert und die am Drüsenteil des Magens befindlichen braun-roten Erosionen gezählt. Bei der Auswertung wurde die Zahl der auf einen Magen kommenden Geschwüre bzw. ihr auf die Zahl der in den Kontrolltiermägen gefundenen Geschwüre bezogenes Verhältnis (Geschwürhemmung) angegeben.

Die Ergebnisse sind in Tabelle 5 zusammengefasst.

TABELLE 5

Hemmung von durch Aspirin hervorgerufenen Magengeschwüren

| Behandlung | N | Dosis (mg/kg p.o.) | Geschwür/Magen | Hemmung (%) |
|---|---|---|---|---|
| Aspirin-Kontrolle | 30 | 100 + Trägermaterial | 15,5 + 3,1 | — |
| Aspirin + A | 20 | 100 + 1 | 9,5 + 4,1 | 38 |
| Aspirin + A | 10 | 100 + 2 | 8,0 + 3,0 | 47* |
| Aspirin + A | 10 | 100 + 10 | 3,7 + 4,5 | 76* |

* $ED_{50}$ 2,1 mg/kg

Aufgrund der obigen Versuchangaben kann festgestellt werden, dass die erfindungsgemässen Verbindungen gastrocytoprotektive Stoffe sind. So zeigt Verbindung A reine gastrocytoprotektive Wirkung, säuresekretionshemmende Wirkung tritt erst bei einer etwa 500mal grösseren Dosis auf. Die cytoprotektive Wirkung wird nicht durch Indomethazin-Vorbehandlung aufgehoben, deshalb muss sie als ein von Prostaglandinen unabhängiger Vorgang betrachtet werden.

Wie bereits erwähnt, können die Verbindungen der allgemeinen Formel I, worin die Bedeutung von R, Z, $R^1$ und $R^2$ die obige ist, durch die Verfahren a) - c) hergestellt werden.

Nach dem Verfahren a) wird die Reaktion der Verbindungen der allgemeinen Formeln II und III in einem Lösungsmittel, vorzugsweise in Gegenwart eines Säurebindemittels durchgeführt. Als Lösungsmittel können zweckmässigerweise Alkohole mit 1-4 Kohlenstoffatomen, Wasser oder ein Gemisch aus ihnen verwendet werden. Als Säurebindemittel werden vorzugsweise Alkalimetall-hydroxyde, -karbonate, -alkoholate oder organische Basen, wie beispielsweise Triäthylamin oder quaternäre Ammoniumverbindungen verwendet. Die Reaktionstemperatur kann sich — abhängig von den Reagenzien, dem angewendeten Lösungsmittel und eventuellen Nebenreaktionen — innerhalb breiter Grenzen bewegen, im Interesse einer entsprechenden Reaktionsgeschwindigkeit ist es jedoch zweckmässig, bei einer Temperatur zwischen 25 °C und 80 °C zu arbeiten. Im Falle der Wahl des entsprechenden Lösungsmittels können die anorganischen Salze am Ende der Reaktion durch Filtern entfernt werden. Nach Eindampfen des Reaktionsgemisches können die kristallinen Produkte durch Umkristallisieren gereinigt werden, die als Base nicht kristallisierenden Stoffe dagegen können nach der Extraktion aus Wasser mit einem in Wasser nicht löslichen organischen Lösungsmittel, z.B. mit chlorierten Kohlenwasserstoffen, mit Äthern oder Äthylacetat durch Eindampfen der organischen Phase abgetrennt werden und gegebenenfalls durch Vakuumdestillation gereinigt werden. Aus den in Form der Base nicht kristallisierenden Produkten können gut kristallisierende Säureadditionssalze, zweckmässigerweise Hydrochloride, gebildet werden.

Das erfindungsgemässe Verfahren a) ist auch in saurem Milieu durchführbar. Dabei ist es zweckmässig, die Reagenzien in konzentrierter wässriger Salzsäurelösung bei einer Temperatur, die dem Siedepunkt des Gemisches entspricht, reagieren zu lassen.

Im Falle des erfindungsgemässen Verfahrens b) wird die Reaktion der Verbindungen der allgemeinen Formeln IV und V unter Bedingungen, die im wesentlichen der ersten Variante des Verfahrens a) entsprechen, in Gegenwart einer Base durchgeführt.

Gemäss dem Verfahren c) lässt man das reaktionsfähige Derivat einer Verbindung der allgemeinen Formel VI — vorzugsweise ein entsprechendes Halogenid — oder eines seiner Säureadditionssalze mit dem Überschuss (vorzugsweise von 2-4 Mol) eines Amins der allgemeinen Formel VII in einem organischen Lösungsmittel, gewünschtenfalls unter Druck, zweckmässigerweise bei einer Temperatur zwischen 50 °C und 150 °C reagieren. Als Lösungsmittel können vorzugsweise Alkohole, chlorierte organische Lösungsmittel und Lösungsmittel vom Säureamid-Typ, z.B. Dimethyl-formamid, verwendet werden.

Das für die Herstellung von Primäraminen geeignete Verfahren d) ($R^1$ und $R^2$ stehen in gleicher Weise für ein Wasserstoffatom) wird under den Bedingungen der Gabriel-Synthese, in einem neutralen organischen Lösungsmittel, vorzugsweise in Dimethylformamid, zweckmässigerweise bei leicht erhöhter Temperatur durchgeführt. Die Entfernung der Phthaloyl-Schutzgruppe erfolgt zweckmässigerweise durch Hydrolyse in Gegenwart einer Base. Als Base kann beispielsweise Hydrazin oder Methylamin verwendet werden.

Die Verbindungen der allgemeinen Formel II sind teils bekannt, so sind sie in der unter der Nr. 0 032 516 offengelegten europäischen Patentanmeldung Nr. 8 010 027.2 beschrieben, teils sind sie durch bekannte chemische Methoden leicht herstellbar [Org. Synth. Coll. Vol. 4, 88 (1963); Wolfenstein und Hartwich, Ber. 48, 2042 (1915)].

Die in Verfahren a) verwendeten Verbindungen der allgemeinen Formel III, in Verfahren b) angewendeten Verbindungen der allgemeinen Formel V und in Verfahren c) verwendeten Verbindungen der allgemeinen Formel VII — wobei die Bedeutung der Substituenten die obige ist — und das Phthalimid-kalium sind bekannte, im Handel erhältliche Stoffe bzw. aus solchen herstellbar.

Die Verbindungen der allgemeinen Formel IV sind teils bekannt (spanische Patentschriften Nr. 506 366, 506 367 und 506 368), teils sind sie auf an sich bekannte Weise aus bekannten Verbindungen herstellbar.

Die neuen Verbindungen der allgemeinen Formel VI und ihre reaktionsfähigen Derivate, so die Halogenide der allgemeinen Formel VIII, sind gemäss EP-A-0 177 054 herstellbar. Die Chloride z.B. können aus 3-Benzoyl-2-(2-hydroxy-äthyl)-thio-pyridin mit Chloriermitteln hergestellt werden, die für den Austausch der Hydroxylgruppe geeignet sind, so z.B. mit Thionylchlorid. Die Reaktion, ausgehend vom Säureadditionssalz der Hydroxyäthyl-Verbindung, kann in einem organischen Lösungsmittel, z.B. in Chloroform, Benzol, Acetonitril, oder in einem dipolaren aprotischen Lösungsmittel durchgeführt werden. Aus dem obigen Ausgangsstoff können mit Sulfonsäurechloriden, z.B. mit p-Toluol-sulfonsäurechlorid bzw. mit Methan-sulfonsäurechlorid, weitere reaktionsfähige Ester bzw. entsprechende Säureadditionssalze, z.B. Hydrochloride, hergestellt werden.

Wie bereits erwähnt, können die Substituenten $R^1$ und/oder $R^2$ in den neuen Verbindungen der allgemeinen Formel I auf an sich bekannte Weise zu anderen Substituenten konvertiert werden, die unter eine früher angegebene Definition von $R^1$ bzw. $R^2$ fallen.

So können die Verbindungen der allgemeinen Formel I, worin $R^1$ und $R^2$ gleichzeitig die Bedeutung eines Wasserstoffatoms besitzen, durch Acylierung zu entsprechenden Verbindungen umgebildet werden, die als $R^1$ und/oder $R^2$ eine Gruppe der allgemeinen Formel $R-\underset{\underset{O}{\|}}{C}-$ (Bedeutung von R ist die früher angegebene) enthalten. Umgekehrt ist von den Verbindungen, die als $R^1$ und/oder $R^2$ die allgemeine Gruppe $R-\underset{\underset{O}{\|}}{C}-$ enthalten, die Acylgruppe auf bekannte Weise, durch Hydrolyse, abtrennbar, was zur Entstehung von entsprechenden Primäraminen führt.

Durch reduktive Kondensation der Primäramine

der allgemeinen Formel I, die sowohl als $R^1$ als auch als $R^2$ ein Wasserstoffatom enthalten, mit den entsprechenden Aldehyden bzw. Ketonen sind Sekundär-, dann durch weitere Reagenzienzugabe Tertiäramine (die Bedeutung von $R^1$ und/oder $R^2$ ist eine Alkylgruppe mit 1-4 Kohlenstoffatomen oder eine Alkyl-phenylgruppe mit 7-11 Kohlenstoffatomen) herstellbar. So sind durch Kondensation eines Aldehyds bzw. Ketons der allgemeinen Formel IX

$$R^1R^2{>}C = O \qquad (IX)$$

und eines Primäramins der allgemeinen Formel I in Gegenwart eines Reduktionsmittels entsprechende $R^1$- und $R^2$-Substituenten enthaltende Sekundär- bzw. Tertiäramine herstellbar. Als Reduktionsmittel verwendet man vorzugsweise Ameisensäure oder eines ihrer Derivate oder z.B. Natrium-borhydrid. Diese Methode ist besonders für die Herstellung von Verbindungen der allgemeinen Formel I geeignet, die als $R^1$ und/oder $R^2$ eine Alkylgruppe mit 1-4 Kohlenstoffatomen oder eine Alkyl-phenylgruppe mit 7-11 Kohlenstoffatomen, vorzugsweise eine Methyl- oder Benzylgruppe, enthalten.

Die durch das erfindungsgemässe Verfahren hergestellten Verbindungen der allgemeinen Formel I können gewünschtenfalls zu Säureadditionssalzen konvertiert werden. Die Säureadditionssalzbildung kann in einem beliebigen neutralen Lösungsmittel, z.B. in einem aliphatischen Alkohol mit 1-6 Kohlenstoffatomen, durchgeführt werden, indem man die Verbindungen der allgemeinen Formel I im obigen Lösungsmittel löst, dann zur Lösung so lange die entsprechende Säure bzw. die mit dem obigen Lösungsmittel hergestellte Lösung der Säure dazugibt, bis der pH des Gemisches auf sauer umschlägt. Danach kann das entstandene Säureadditionssalz aus dem Reaktionsgemisch auf eine geeignete Weise, z.B. durch Filtern, abgetrennt werden.

Der Wirkstoff der allgemeinen Formel I kann, vermischt mit in der Pharmazie gebräuchlichen, für parenterale oder enterale Dosierung geeigneten, nicht toxischen, inerten festen oder flüssigen Trägerstoffen und/oder Hilfsstoffen, zu pharmazeutischen Präparaten verarbeitet werden. Als Trägerstoffe können z.B. Wasser, Gelatine, Laktose, Milchzucker, Stärke, Pektin, Magnesiumstearat, Stearinsäure, Talcum, Pflanzenöle (wie Erdnussöl, Olivenöl) usw., verwendet werden. Die Wirkstoffe können in gebräuchlicher Form von pharmazeutischen Präparaten, so in Form von runden oder eckigen Tabletten, Dragees, Kapseln (wie Gelatinekapseln), Pillen, Zäpfchen usw., bereitet werden. Die Menge des festen Trägerstoffes kann sehr verschieden sein, vorzugsweise ist es ein Wert zwischen etwa 25 mg und 1 g. Die Präparate können gegebenenfalls die üblichen Hilfsstoffe enthalten, z.B. Konservierungsmittel, Stabilisierungsmittel, Netzmittel, Emulgierungsmittel usw. Ihre Herstellung kann mittels üblicher Methoden. z.B. im Falle von festen Präparaten durch Sieben, Mischen, Granulieren und Pressen der Komponenten, vor sich gehen. Die Präparate können weiteren gebräuchlichen pharmakotechnischen Operationen, z.B. Sterilisierung, unterworfen werden.

Die Erfindung wird anhand der nachstehenden Beispiele erläutert, ohne den Schutzumfang auf diese Beispiele einzuschränken.

Die durch die allgemeinen Formel I gekennzeichneten Verbindungen sind nach der Nomenklatur der Chemikal Abstracts substituierte Methanone oder Säureamide, den durch die Benennung hervorgehobenen funktionellen Gruppen entsprechend. Der Einfachheit und Überschaubarkeit halber werden in jedem der folgenden Beispiele die Verbindungen — aufgrund des in jeder von ihnen vorkommenden Pyridinringes — als substituierte Pyridine erwähnt, es wird jedoch auch die Benennung nach der Nomenklatur der Chemical Abstracts angegeben.

*Beispiel 1*

2-[(2-Amino-äthyl)-thio]-3-benzoyl-pyridin · HCl
{2-([2-Amino-äthyl]-thio)-3-pyridinyl}-phenyl-
-methanon · HCl

a) In die mit 100,0 $cm^3$ Äthanol bereitete heisse Lösung von 10,88 g (0,05 Mol) 3-Benzoyl-2-chlor-pyridin und 8,51 g (0,075 Mol) Cysteamin · HCl wird innerhalb von 30 Minuten mit 50,0 $cm^3$ Äthanol bereitete Lösung von 9,88 g (0,15 Mol) 85,0%-igem Kaliumhydroxyd getropft. Das Reaktionsgemisch wird weitere 90 Minuten gekocht, mit konzentrierter Salzsäure neutralisiert, das anorganische Salz wird ausgefiltert und die Lösung eingedampft. Der Rest wird in Wasser gelöst, erst bei einem pH-Wert von 4, dann von 11 mit 1,2-Dichloräthan extrahiert, dann wird der letztere Extrakt mit Wasser gewaschen und eingedampft. Aus dem öligen Destillationsrest wird mit salzsaurem Äthanol ein Salz gebildet, dann aus dem Äthanol umkristallisiert. Der Schmelzpunkt der so hergestellten 9,90 g (67,2%) 2-[(2-Amino-äthyl)-thio]-3-benzoyl-pyridin · HCl beträgt 190 - 192°C.

b) Die Reaktion zwischen 3-Benzoyl-2-chlorpyridin und Cysteamin-hydrochlorid in konzentrierter wässriger Salzsäurelösung durchgeführt, erhält man ebenfalls 2-[(2-Amino-äthyl)-thio]-3-benzoyl-pyridin · HCl, dessen Schmelzpunkt bei 190 - 191°C liegt.

c) 1,60 g (0,04 Mol) Natriumhydroxyd werden in 25,0 ml Wasser gelöst, dann werden 4,31 g (0,02 Mol) 3-Benzoyl-1,2-dihydro-pyridin-2-thion und 2,32 g (0,02 Mol) 2-Chlor-äthyl-amin · HCl dazugegeben, und das Reaktionsgemisch wird 40 Minuten lang gekocht. Nach Abkühlen wird das Gemisch bei einem pH-Wert von 4, dann von 11 mit 1,2-Dichloräthan, dann die letztere Dichloräthan-Phase mit wässriger Salzsäure extrahiert. Die wässrige Phase wird eingedampft und das kristalline Rohprodukt aus Äthanol umkristallisiert. Der Schmelzpunkt des so erhaltenen 2-[(2-Amino-äthyl)-thio]-3-benzoyl-pyridin · HCl beträgt 190 - 191°C.

Analysenergebnisse, auf der Summenformel $C_{14}H_{14}N_2OS \cdot HCl$ (Mg.: 294,80) basierend:

errechnet:
C 57,04 H 5,13 Cl 12,02 N 9,50 S 10,88%
gefunden:
C 56,94 H 5,22 Cl 11,57 N 9,67 S 10,72%

IR-Spektrum (KBr)
1648 $cm^{-1}$, $>C=O$;

1284 $cm^{-1}$, $-S-CH_2-$;
1598,788, 758, 701 $cm^{-1}$, -Ar;
|
3200-2300 $cm^{-1}$, $-NH^+$;
|

NMR-Spektrum (DMSO $d_6$)
3,13 ppm, m, $-S-CH_2-$;
3,37 ppm, m, $-N-CH_2-$;
7,1 ppm, 2xd, Pyridin 4,5-H;
7,2-7,7 ppm, m, Phenylring;
8,11 ppm, b;
8,4 ppm, 2xd, Pyridin 6-H.

*Beispiel 2*

3-Benzoyl-2-{(2-[N,N-diacetyl-amino]-äthyl)-thio}-
-pyridin
N-Acetyl-N-{2-([3-benzoyl-2-pyridinyl]-thio)-
-äthyl}-acetamid

16,54 g (0,056 Mol) 2-([2-Amino-äthyl]-thio)-3--benzoyl-pyridin · HCl werden in Wasser gelöst. Die Lösung wird alkalisiert, die Base mit Äthylacetat extrahiert, dann eingedampft. Zum Rest werden 50 $cm^3$ (54,1 g; 0,53 Mol) Essigsäureanhydrid gegeben, und das Reaktionsgemisch wird 30 Minuten lang gekocht. Danach wird der Anhydrid-Überschuss mit 50 $cm^3$ Wasser vernichtet und die Lösung eingedampft. Der kristalline Rest wird mit Wasser verrieben, gefiltert, dann aus Äthanol umkristallisiert. Der Schmelzpunkt des erhaltenen 3-Benzoyl--2-{(2-[N,N-diacetyl-amino]-äthyl)-thio}-pyridin beträgt 104 - 105°C.

Analysenergebnisse, auf der Summenformel $C_{18}H_{18}O_3NS$ (Mg.: 342,41) basierend:

errechnet: C 63,14 H 5,30 N 8,18 S 9,36%
gefunden: C 63,21 H 5,56 N 8,16 S 9,21%

IR-Spektrum (KBr)
1704, 1692 $cm^{-1}$, >C=O Amid;
1642 $cm^{-1}$, >C=O Keton;
1598, 787, 753, 708 $cm^{-1}$, -Ar;

NMR-Spektrum ($CDCl_3$)
2,5 ppm, s, $-Ac-CH_3$;
3,4 ppm, m, $-S-CH_2-$;
4,0 ppm, m, $-N-CH_2-$;
7,2 ppm, 2xd, Pyridin 4,5-H;
7,6 ppm, m, Phenylring;
8,7 ppm, m, Pyridin 6-H.

*Beispiel 3*

2-([2-Amino-äthyl]-thio)-3-[p-chlor-benzoyl]-pyri-
din · HCl
2-{([Amino-äthyl]-thio)-3-pyridinyl}-[4-chlor-
-phenyl]-methanon · HCl

In die mit 30 $cm^3$ Äthanol bereitete Lösung von 16,2 g (0,05 Mol) 2-Chlor-3-[p-chlor-benzoyl]-pyridin und 8,52 g (0,075 Mol) Cysteamin · HCl werden bei Zimmertemperatur innerhalb von 30 Minuten mit 35 $cm^3$ Äthanol bereitete Lösung von 9,9 g (0,15 Mol) 85%igem Kaliumhydroxyd getropft. Die Suspension wird 23 Stunden lang gerührt, dann mit konzentrierter wässriger Salzsäurelösung neutralisiert. Das anorganische Salz wird herausgefiltert, die äthanolische Lösung hingegen eingedampft. Der feste Rest wird in Wasser gelöst, bis zu einem pH von 10 alkalisiert und mit Äthylacetat extrahiert. Die Äthylacetat-Phase wird eingedampft, aus der zurückbleibenden öligen Base wird mit salzsaurem Isopropanol ein Salz gebildet, gefiltert, getrocknet, dann aus Nitromethan umkristallisiert. Der Schmelzpunkt des erhaltenen 2-([2-Amino-äthyl]-thio)-3-[p-chlor-benzoyl]-pyridinn · HCl liegt bei 171 - 173°C.

Analysenergebnisse, auf der Summenformel $C_{14}H_{13}ClN_2OS \cdot HCl$ (Mg.: 329,24) basierend:

errechnet: C 51,07 H 4,29 N 8,51 S 9,74%
gefunden: C 50,94 H 4,17 N 8,70 S 9,46%

IR-Spektrum (KBr)
3250-2300 $cm^{-1}$, $-N^+H$;
1655 $cm^{-1}$, >C=O;
1088 $cm^{-1}$, -Ar-Cl;
1590, 842, 815, 765 $cm^{-1}$, -Ar;

NMR-Spektrum ($CDCl_3$ + DMSO $d_6$)
3,4 ppm, m, $-S-CH_2-$ und $-N-CH_2-$;
7,5 ppm, m, Pyridin 4,5-H und Phenylring;
8,7 ppm, 2xd, Pyridin 6-H.

*Beispiel 4*

2-([2-Amino-äthyl]-thio)-3-[2,5-dimethyl-benzoyl]-
-pyridin · HCl
{2-([2-Amino-äthyl]-thio)-3-pyridinyl}[2,5-di-
methyl-phenyl]methanon · HCl

In die mit 200 $cm^3$ Äthanol bereitete heisse Lösung von 24,6 g (0,1 Mol) 3-[2,5-Dimethyl-benzoyl]--2-chlor-pyridin und 17,04 g (0,15 Mol) Cysteamin · HCl wird innerhalb von 30 Minuten mit 100 $cm^3$ Äthanol bereitete Lösung von 19,8 g (0,3 Mol) 85%igem Kaliumhydroxyd gegeben und das Gemisch weitere 20 Minuten lang gekocht, dann mit 450 $cm^3$ Wasser verdünnt. Der pH-Wert der Lösung wird auf 2 eingestellt, und es wird mit Diisopropyl--äther extrahiert, dann wird der pH auf 11 eingestellt und mit Äthylacetat extrahiert. Die Äthylacetat-Phase wird eingedampft, aus dem öligen Rest wird mit salzsaurem Isopropanol Salz gebildet, gefiltert, dann aus Isopropanol umkristallisiert. Man erhält 16,07 g (49,7%) 2-([2-Amino-äthyl]-thio)-3-[2,5-dimethyl--benzoyl]-pyridin · HCl, dessen Schmelzpunkt 208 bis 210°C beträgt.

Analysenergebnisse, auf der Summenformel $C_{16}H_{18}N_2OS \cdot HCl$ (Mg.: 322,85) basierend:

errechnet: C 59,53 H 5,93 N 8,68 S 9,93%
gefunden: C 59,70 H 6,10 N 8,74 S 9,77%

IR-Spektrum (KBr)
1660 $cm^{-1}$, >C=O;
3250-2400 $cm^{-1}$, $-N^+H$;
1580, 810, 770 $cm^{-1}$, -Ar;

NMR-Spektrum ($CDCl_3$)
2,3 ppm, s, $-Ar-CH_3$;
3,6 ppm, s, $-S-CH_2-$ und $-N-CH_2-$;
7,3 ppm, m, Pyridin 4,5-H und Phenyl 3,4,6-H;
7,8 ppm, 2xd, Pyridin 6-H;
8,9 ppm, xb; $>N^+H_3$.

*Beispiel 5*

2-([2-Amino-äthyl]-thio)-2-benzoyl-6-propyl-pyridin · 2 HCl

{2-([2-Amino-äthyl]-thio)-6-propyl-4-pyridinyl}phenyl-methanon · 2 HCl

In die mit 60 $cm^3$ Äthanol bereitete heisse Lösung von 23,37 g (0,09 Mol) 4-Benzoyl-2-chlor-6-propyl--pyridin und 20,43 g (0,18 Mol) Cysteamin · HCl wird mit 90 $cm^3$ Äthanol bereitete Lösung von 22,4 g (0,34 Mol) 85%igem Kaliumhydroxyd getropft, die Suspension wird 4,5 Stunden lang gekocht, dann mit 500 $cm^3$ Wasser verdünnt. Die Lösung wird mit konzentrierter Salzsäure bis zu einem pH-Wert von 1 angesäuert, mit Diisopropyläther extrahiert, dann wird die wässrige Phase bis zu einem pH von 10 alkalisiert und mit Äthylacetat extrahiert. Die Äthylacetat-Phase wird eingedampft, aus der zurückbleibenden öligen Base wird mit Äthylacetat Salz gebildet, dann aus n-Butanol umkristallisiert. Das erhaltene 2-([2--Aminoäthyl]-thio)-4-benzoyl-6-propyl-pyridin · 2 HCl schmilzt bei 185°C.

Analysenergebnisse, auf der Summenformel $C_{17}H_{20}N_2OS \cdot 2HCl$ (Mg.: 373,33) basierend:

errechnet: C 54,69 H 5,94 N 7,50 Cl 18,99%
gefunden: C 54,65 H 5,78 N 7,58 Cl 18,58%

IR-Spektrum (KBr)
3200-2100 $cm^{-1}$, $\geqslant N^+H$;
1664 $cm^{-1}$, >C=O;
1286 $cm^{-1}$, -S-$CH_2$-;
1598, 802, 721, 692 $cm^{-1}$, -Ar;

NMR-Spektrum ($D_2O$)
1,3 ppm, t, Pr-$CH_3$;
2,0 ppm, q, $CH_2$-$CH_2$-$CH_3$;
3,2 ppm, t, Ar-$CH_2$-R;
3,9 ppm, m, -S-$CH_2$- und -N-$CH_2$;
8,0 ppm, m, Ar-H.

*Beispiel 6*

2-([2-N,N-Dimethyl-amino-äthyl]-thio)-3-benzoyl--pyridin · HCl

2-{([2-N,N-Dimethyl-amino-äthyl]-thio)-3-pyridinyl}-phenylmethanon · HCl

14,75 g (0,05 Mol) 2-([2-Amino-äthyl]-thio)-3--benzoyl-pyridin · HCl werden in Wasser gelöst. Die Lösung wird alkalisiert und mit Äthylacetat ausgeschüttelt. Die Äthylacetat-Extrakte werden eingedampft, zum Rest werden 13,5 g 85%ige Ameisensäure, dann 9,4 g 35%iges Formalin gegeben und 20 Stunden lang gekocht. Danach werden 4,3 $cm^3$ konzentrierte Salzsäure zum Gemisch gegeben, und es wird eingedampft. Der Rest wird in Wasser gelöst, alkalisiert, mit Äthylacetat ausgeschüttelt und getrocknet. Durch Einführen von Salzsäuregas wird das entsprechende Hydrochlorid abgetrennt, dann wird aus Methyl-äthylketon umkristallisiert. Der Schmelzpunkt des erhaltenen 2-([2-N,N-Dimethyl--amino-äthyl]-thio)-3-benzoyl-pyridin · HCl beträgt 146 - 148°C.

Analysenergebnisse, auf der Summenformel $C_{16}H_{19}ClN_2OS$ (Mg.: 322,86) basierend:

errechnet:
C 59,52 H 5,93 Cl 10,98 N 8,68 S 9,93%
gefunden:
C 59,58 H 5,58 Cl 11,11 N 8,75 S 9,69%

IR-Spektrum (KBr)
2800-2000 $cm^{-1}$, $\geqslant N^+H$;
2810 $cm^{-1}$, >N-$CH_3$;
1650 $cm^{-1}$, >C=O;
1280 $cm^{-1}$, -S-$CH_2$-;
1588, 800, 760, 712 $cm^{-1}$, -Ar;

NMR-Spektrum ($D_2O$)
3,3 ppm, s, $\geqslant$N-$CH_3$;
3,7 ppm, s, -S-$CH_2$ + N-$CH_2$;
7,3-7,9 ppm, m, Phenylring und Pyridin 4,5-H;
8,9 ppm, 2xd, Pyridin6-H.

*Beispiel 7*

N-[1-Phenyl-2-propyl]-2-([2-amino-äthyl]-thio)--3-benzoyl-pyridin

1,32 g (0,02 Mol) 85%iges Kaliumhydroxyd werden in 8 $cm^3$ Methanol gelöst und die mit 20 $cm^3$ Methanol bereitete Lösung von 5,9 g (0,02 Mol) 2-([2-Amino-äthyl]-thio)-3-benzoyl-pyridin-hydrochlorid und 2,64 $cm^3$ Benzyl-methylketon dazugetröpfelt. Darauffolgend gibt man bei 40°C innerhalb einer Stunde in kleinen Teilen 0,19 g (0,0005 Mol) Natrium-borhydrid zum Gemisch. Das Reaktionsgemisch wird weitere 10 Stunden lang gemischt, dann erfolgt nach Eindampfen und Chloroform-Extraktion ein erneutes Eindampfen und Äther-Auslösen. Das Gewicht des in Äther gelösten Stoffes beträgt 3,41 g, er wird unter Nitromethan kristallin. Nach Umkristallisieren aus Acetonitril erhält man 0,7 g der Verbindung laut Titel, ihr Schmelzpunkt liegt bei 92°C.

Analysenergebnisse, auf der Summenformel $C_{23}H_{26}N_2OS$ (Mg.: 378,53) basierend:

errechnet: C 72,97 H 6,92 N 7,40 S 8,47%
gefunden: C 72,90 H 7,00 N 7,25 S 8,90%

IR-Spektrum (KBr)
3300-2200 $cm^{-1}$, OH + NH;
1082 $cm^{-1}$, C-OH;
1591, 1570 $cm^{-1}$, Ar Gerüst;
783, 748, 700 $cm^{-1}$, Ar H Def.;

NMR-Spektrum ($CDCl_3$)
1,1 ppm, d, -$CH_3$;
2,5-3,4 ppm, m, { Ar-$CH_2$, S-$CH_2$, N-$CH_2$, N-CH;
6,0 ppm, s, O-CH;
6,8-8,3 ppm, m, Ar-H.

**Patentansprüche für die Vertragsstaaten:**
BE, CH, DE, FR, GB, IT, LI, NL, SE

1. 2-Pyridin-thiol-Derivate der allgemeinen Formel I

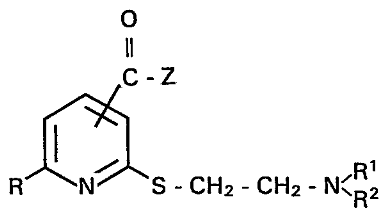

(I)

worin

R für ein Wasserstoffatom oder eine Alkylgruppe mit 1-4 Kohlenstoffatomen steht,

Z für eine gegebenenfalls durch ein oder mehrere Halogenatome und/oder für eine durch eine Alkylgruppe mit 1-4 Kohlenstoffatomen substituierte Phenylgruppe steht,

$R^1$ und $R^2$ unabhängig voneinander die Bedeutung eines Wasserstoffatoms, einer Alkylgruppe mit 1-4 Kohlenstoffatomen, einer Alkyl-phenylgruppe mit 7-11 Kohlenstoffatomen oder einer Gruppe der allgemeinen Formel R-C- besitzen, wobei die Bedeutung
||
O
von R die obige ist,

und der Substituent -C-Z bindet sich an die 3- oder
||
O
4-Stelle des Pyridinringes,

mit der Festlegung, dass falls sowohl $R^1$ als auch $R^2$ die Bedeutung einer Methylgruppe besitzen, und die Bedeutung von Z eine 4-Chlor-phenylgruppe ist, R nicht Wasserstoffatom bedeutet,

und ihre Säureadditionssalze.

2. 2-([2-Amino-äthyl]-thio)-3-benzoyl-pyridin und sein Hydrochlorid.

3. 3-Benzoyl-2-{(2-[N,N-diacetyl-amino]-äthyl)-thio}-pyridin.

4. Pharmazeutisches Präparat, dadurch gekennzeichnet, dass es als Wirkstoff mindestens eine Verbindung der allgemeinen Formel I, worin R, Z, $R^1$ und $R^2$ die in Anspruch 1 angegebene Bedeutung besitzen, oder deren therapeutisch akzeptierbares Säureadditionssalz neben üblichen Trägerstoffen enthält.

5. Verfahren zur Herstellung von 2-Pyridin-thiol-Derivaten der allgemeinen Formel I

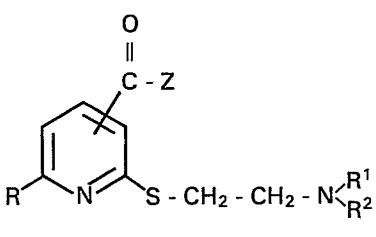

(I)

worin

R für ein Wasserstoffatom oder eine Alkylgruppe mit 1-4 Kohlenstoffatomen steht,

Z für eine gegebenenfalls durch ein oder mehrere Halogenatome und/oder für eine durch eine Alkylgruppe mit 1-4 Kohlenstoffatomen substituierte Phenylgruppe steht,

$R^1$ und $R^2$ unabhängig voneinander die Bedeutung eines Wasserstoffatoms, einer Alkylgruppe mit 1-4 Kohlenstoffatomen, einer Alkyl-phenylgruppe mit 7-11 Kohlenstoffatomen oder einer Gruppe der allgemeinen Formel R-C- besitzen, wobei die Bedeutung
||
O
von R die obige ist,

und der Substituent -C-Z bindet sich an die 3- oder
||
O
4-Stelle des Pyridinringes,

mit der Festlegung, dass falls sowohl $R^1$ als auch $R^2$ die Bedeutung einer Methylgruppe besitzen, und die Bedeutung von Z eine 4-Chlor-phenylgruppe ist, R nicht Wasserstoffatom bedeutet,

und ihrer Säureadditionssalze, dadurch gekennzeichnet, dass

a) ein 2-Halogen-pyridin-Derivat der allgemeinen Formel II

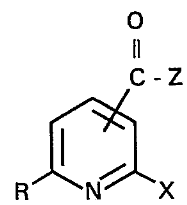

(II)

worin die Bedeutung von R und Z die im Oberbegriff angegebene ist und X für ein Halogenatom steht,

mit einer Thiolverbindung der allgemeinen Formel III

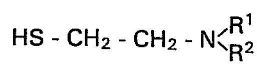

(III)

worin die Bedeutung von $R^1$ und $R^2$ die im Oberbegriff angegebene ist,
oder mit deren Säureadditionssalz umgesetzt wird; oder

b) ein Pyridin-2-thion-Derivat der allgemeinen Formel IV

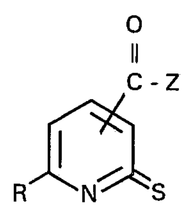

(IV)

worin die Bedeutung von R und Z die im Oberbegriff angegebene ist,

mit einem 2-Halogen-äthyl-amin-Derivat der allgemeinen Formel V

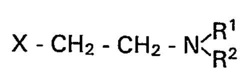

(V)

wobei die Bedeutung von $R^1$ und $R^2$ im Oberbegriff angegeben, die von X Halogenatom ist,

oder dessen Säureadditionssalz umgesetzt wird; oder

c) das reaktionsfähige Derivat einer 2-Hydroxy-äthyl-thio-pyridin-Verbindung der allgemeinen Formel VI

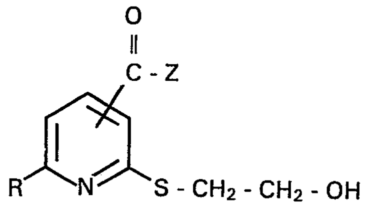

(VI)

worin die Bedeutung von R und Z die im Oberbegriff angegebene ist,

mit einem Amin der allgemeinen Formel VII

$HN{<}^{R^1}_{R^2}$ (VII)

worin $R^1$ und $R^2$ die im Oberbegriff angegebene Bedeutung besitzen,

umgesetzt wird;

d) zur Herstellung von Verbindungen der allgemeinen Formel I, worin sowohl $R^1$ als auch $R^2$ für ein Wasserstoffatom stehen, Z und R die im Oberbegriff angegebene Bedeutung haben,

ein 2-Halogen-äthyl-thio-pyridin-Derivat der allgemeinen Formel VIII

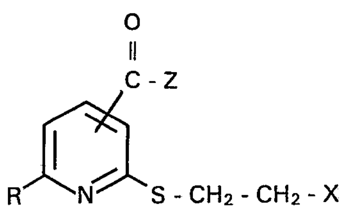

(VIII)

worin die Bedeutung von R und Z die im Oberbegriff angegebene, die von X Halogenatom ist,

mit Phthalimid-kalium umgesetzt wird, dann die Phthaloyl-Schutzgruppe auf an sich bekannte Weise entfernt wird, und

gewünschtenfalls wird der $R^1$- oder $R_2$-Substituent einer im Verfahren a), b), c) oder d) hergestellten Verbindung der allgemeinen Formel I zu einem anderen $R_1$- bzw. $R^2$-Substituenten konvertiert, der unter eine im Oberbegriff gegebene Definition fällt, und/oder

gewünschtenfalls wird eine erhaltene Verbindung der allgemeinen Formel I zu ihrem Säureadditionssalz konvertiert oder aus ihrem Säureadditionssalz freigesetzt.

6. Verfahren nach Anspruch 5, Variante a), dadurch gekennzeichnet, dass die Reaktion in einem Lösungsmittel, in Gegenwart eines Säurebindemittels durchgeführt wird.

7. Verfahren nach Anspruch 6, dadurch gekennzeichnet, dass als Lösungsmittel ein Alkohol mit 1-4 Kohlenstoffatomen und als Säurebindemittel Alkalimetall-hydroxyd verwendet wird.

8. Verfahren nach Anspruch 5, Variante a), dadurch gekennzeichnet, dass die Reaktion in konzentrierter wässriger Salzsäurelösung bei einer Temperatur, die dem Siedepunkt des Gemisches entspricht, durchgeführt wird.

9. Verfahren nach Anspruch 5, Variante b), dadurch gekennzeichnet, dass die Reaktion in einem Alkohol mit 1-4 Kohlenstoffatomen, in Gegenwart von Alkalimetall-hydroxyd durchgeführt wird.

10. Verfahren nach Anspruch 5, Variante c), dadurch gekennzeichnet, dass als reaktionsfähiges Derivat einer Verbindung der allgemeinen Formel VI ein Halogenid der allgemeinen Formel VIII verwendet wird, wobei die Bedeutung von R, Z und X die in Anspruch 5 angegebene ist.

**Patentansprüche für den Vertragsstaat:** AT

1. Verfahren zur Herstellung von 2-Pyridin-thiol-Derivaten der allgemeinen Formel I

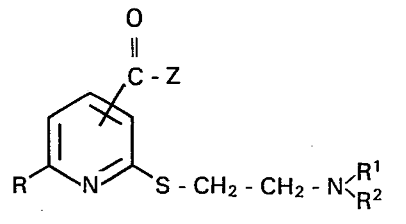

(I)

worin

R für ein Wasserstoffatom oder eine Alkylgruppe mit 1-4 Kohlenstoffatomen steht,

Z für eine gegebenenfalls durch ein oder mehrere Halogenatome und/oder für eine durch eine Alkylgruppe mit 1-4 Kohlenstoffatomen substituierte Phenylgruppe steht,

$R^1$ und $R^2$ unabhängig voneinander die Bedeutung eines Wasserstoffatoms, einer Alkylgruppe mit 1-4 Kohlenstoffatomen, einer Alkyl-phenylgruppe mit 7-11 Kohlenstoffatomen oder einer Gruppe der allgemeinen Formel $R-\underset{\underset{O}{\|}}{C}-$ besitzen, wobei die Bedeutung von R die obige ist,

und der Substituent $-\underset{\underset{O}{\|}}{C}-Z$ bindet sich an die 3- oder 4-Stelle des Pyridinringes,

mit der Festlegung, dass falls sowohl $R^1$ als auch $R^2$ die Bedeutung einer Methylgruppe besitzen, und die Bedeutung von Z eine 4-Chlor-phenylgruppe ist, R nicht Wasserstoffatom bedeutet,

und ihrer Säureadditionssalze, dadurch gekennzeichnet, dass

a) ein 2-Halogen-pyridin-Derivat der allgemeinen Formel II

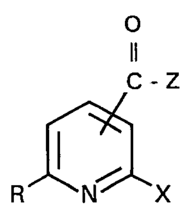

(II)

worin die Bedeutung von R und Z die im Oberbegriff angegebene ist und X für ein Halogenatom steht,

mit einer Thiolverbindung der allgemeinen Formel III

$HS-CH_2-CH_2-N{<}^{R^1}_{R^2}$ (III)

worin die Bedeutung von $R^1$ und $R^2$ die im Oberbegriff angegebene ist,

oder mit deren Säureadditionssalz umgesetzt wird; oder

b) ein Pyridin-1-thion-Derivat der allgemeinen Formel IV

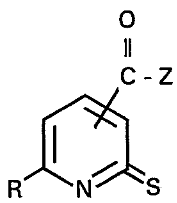

(IV)

worin die Bedeutung von R und Z die im Oberbegriff angegebene ist,

mit einem 2-Halogen-äthyl-amin-Derivat der allgemeinen Formel V

$$X - CH_2 - CH_2 - N\begin{matrix}R^1\\R^2\end{matrix} \qquad (V)$$

wobei die Bedeutung von $R^1$ und $R^2$ im Oberbegriff angegeben ist, die von X Halogenatom ist,

oder dessen Säureadditionssalz umgesetzt wird; oder

c) das reaktionsfähige Derivat einer 2-Hydroxy--äthyl-thio-Verbindung der allgemeinen Formel VI

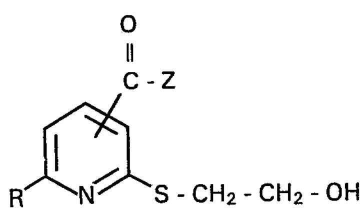

(VI)

worin die Bedeutung von R und Z die im Oberbegriff angegebene ist,

mit einem Amin der allgemeinen Formel VII

$$HN\begin{matrix}R^1\\R^2\end{matrix} \qquad (VII)$$

worin $R_1$ und $R^2$ die im Oberbegriff angegebene Bedeutung besitzen,

umgesetzt wird;

d) zur Herstellung von Verbindungen der allgemeinen Formel I, worin sowohl $R^1$ als auch $R^2$ für ein Wasserstoffatom stehen, Z und R die im Oberbegriff angegebene Bedeutung haben,

ein 2-Halogen-äthyl-thio-pyridin-Derivat der allgemeinen Formel VIII

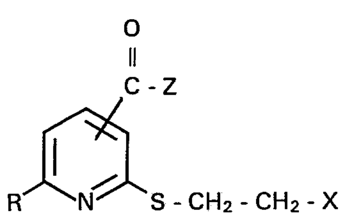

(VIII)

worin die Bedeutung von R und Z die im Oberbegriff angegebene, die von X Halogenatom ist,

mit Phthalimid-kalium umgesetzt wird, dann die Phthaloyl-Schutzgruppe auf an sich bekannte Weise entfernt wird, und

gewünschtenfalls wird der $R^1$- oder $R^2$-Substituent einer im Verfahren a), b), c) oder d) hergestellten Verbindung der allgemeinen Formel I zu einem anderen $R^1$- bzw. $R^2$-Substituenten konvertiert, der unter eine im Oberbegriff gegebene Definition fällt, und/oder

gewünschtenfalls wird eine erhaltene Verbindung der allgemeinen Formel I zu ihrem Säureadditionssalz konvertiert oder aus ihrem Säureadditionssalz freigesetzt.

2. Verfahren nach Anspruch 1, Variante a), dadurch gekennzeichnet, dass die Reaktion in einem Lösungsmittel, in Gegenwart eines Säurebindemittels durchgeführt wird.

3. Verfahren nach Anspruch 2, dadurch gekennzeichnet, dass als Lösungsmittel ein Alkohol mit 1-4 Kohlenstoffatomen und als Säurebindemittel Alkalimetall-hydroxyd verwendet wird.

4. Verfahren nach Anspruch 1, Variante a), dadurch gekennzeichnet, dass die Reaktion in konzentrierter wässriger Salzsäurelösung bei einer Temperatur, die dem Siedepunkt des Gemisches entspricht, durchgeführt wird.

5. Verfahren nach Anspruch 1, Variante b), dadurch gekennzeichnet, dass die Reaktion in einem Alkohol mit 1-4 Kohlenstoffatomen, in Gegenwart von Alkalimetall-hydroxyd durchgeführt wird.

6. Verfahren nach Anspruch 1, Variante c), dadurch gekennzeichnet, dass als reaktionsfähiges Derivat einer Verbindung der allgemeinen Formel VI ein Halogenid der allgemeinen Formel VIII verwendet wird, wobei die Bedeutung von R, Z und X die in Anspruch 5 angegebene ist.

7. Verfahren zur Herstellung eines pharmazeutischen Präparates, dadurch gekennzeichnet, dass man die gemäss Anspruch 1 bis 6 hergestellten Verbindungen oder ihre pharmazeutisch annehmbaren Additionssalze als Wirkstoffe mit entsprechenden Exzipienten formuliert.

**Claims for the Contracting States:**
BE, CH, DE, FR, GB, IT, LI, NL, SE

1. 2-Pyridine-thiol derivatives of the general formula I

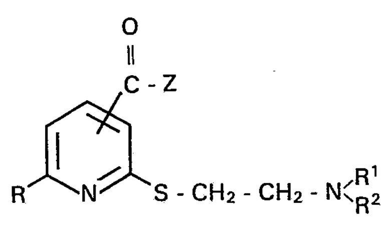

(I)

in which

R represents a hydrogen atom or an alkyl group having 1-4 carbon atoms,

Z represents a phenyl group which is optionally substituted by one or more halogen atoms and/or represents a phenyl group which is substituted by an alkyl group having 1-4 carbon atoms,

$R^1$ and $R^2$ have, independently of one another, the

meaning of a hydrogen atom, an alkyl group having 1-4 carbon atoms, an alkyl-phenyl group having 7-11 carbon atoms or a group of the general formula $R-\overset{\overset{O}{\|}}{C}-$ , the meaning of R being the above, and the substituent $-\overset{\overset{O}{\|}}{C}-Z$ is bonded to the 3 or 4 position of the pyridine ring, with the delimitation that if both $R^1$ and $R^2$ have the meaning of a methyl group, and the meaning of Z is a 4-chloro-phenyl group, R does not denote hydrogen atom

and their acid addition salts.

2. 2-([2-Amino-ethyl]-thio)-3-benzoyl-pyridine and its hydrochloride.

3. Benzoyl-2-{(2-[N,N-diacetyl-amino]-ethyl)--thiol}-pyridine.

4. Pharmaceutical composition characterized in that it contains as active ingredient at least one compound of the general formula I in which R, Z, $R^1$ and $R^2$ have the meaning indicated in Claim 1, or its therapeutically acceptable acid addition salt, besides customary vehicles.

5. Process fot the preparation of 2-pyridine-thiol derivatives of the general formula I

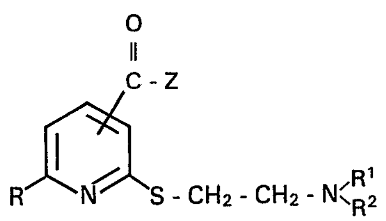

(I)

in which

R represents a hydrogen atom or an alkyl group having 1-4 carbon atoms,

Z represents a phenyl group which is optionally substituted by one or more halogen atoms and/or represents a phenyl group which is substituted by an alkyl group having 1-4 carbon atoms,

$R^1$ and $R^2$ have, independently of one another, the meaning of a hydrogen atom, an alkyl group having 1-4 carbon atoms, an alkyl-phenyl group having 7-11 carbon atoms or a group of the general formula $R-\overset{\overset{O}{\|}}{C}-$ , the meaning of R being the above, and the substituent $-\overset{\overset{O}{\|}}{C}-Z$ is bonded to the 3 or 4 position of the pyridine ring, with the delimitation that if both $R^1$ and $R^2$ have the meaning of a methyl group, and the meaning of Z is a 4-chloro-phenyl group, R does not denote hydrogen atom

and their acid addition salts, characterized in that,

a) a 2-halogeno-pyridine derivative of the general formula II

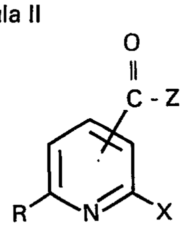

(II)

in which the meaning of R and Z is that indicated in the preamble and X represents a halogen atom

is reacted with a thiol compound of the general formula III

$$HS - CH_2 - CH_2 - N\begin{matrix}\diagup R^1\\ \diagdown R^2\end{matrix} \qquad (III)$$

in which the meaning of $R^1$ and $R^2$ is that indicated in the preamble

or its acid addition salt; or

b) a pyridine-2-thione derivative of the general formula IV

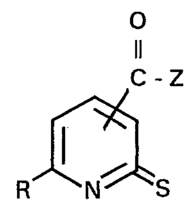

(IV)

in which the meaning of R and Z is that indicated in the preamble

is reacted with a 2-halogeno-ethyl-amine derivative of the general formula V

$$X - CH_2 - CH_2 - N\begin{matrix}\diagup R^1\\ \diagdown R^2\end{matrix} \qquad (V)$$

in which the meaning of $R^1$ and $R^2$ is that indicated in the preamble, and that of X is halogen atom

or its acid addition salt; or

c) the reactive derivative of a 2-hydroxy-ethyl--thio-pyridine compound of the general formula VI

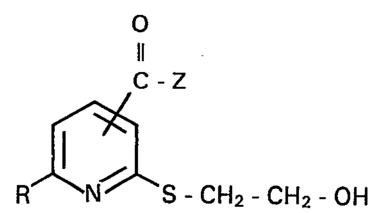

(VI)

in which the meaning of R and Z is that indicated in the preamble

is reacted with an amine of the general formula VII

$$HN\begin{matrix}\diagup R^1\\ \diagdown R^2\end{matrix} \qquad (VII)$$

and which $R^1$ and $R^2$ have the meaning indicated in the preamble; or

d) for the preparation of compounds of the general formula I in which $R^1$ and $R^2$ both represent a hydrogen atom, and Z and R have the meaning indicated in the preamble,

a 2-halogeno-ethyl-thio-pyridine derivative of the general formula VIII

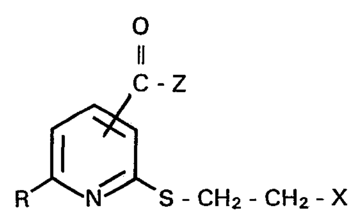

(VIII)

in which the meaning of R and Z is that indicated in the preamble, and that of X is halogen atom

is reacted with potassium phthalimide, and the phthaloyl protective group is removed in a manner known per se, and, if desired the $R^1$ or $R^2$ substituent of a compound of the general formula I prepared in process a), b), c) or d) is converted into another $R^1$ or $R^2$ substituent which falls under a definition given in the preamble, and/or, if desired, a resulting compound of the general formula I is converted into its acid addition salt or is liberated from its acid addition salt.

6. Process according to Claim 5, variant a), characterized in that the reaction is carried out in a solvent in the presence of an acid-binding agent.

7. Process according to Claim 6, characterized in that an alcohol having 1-4 carbon atoms is used as solvent, and alkali metal hydroxide is used as acid-binding agent.

8. Process according to Claim 5, variant a), characterized in that the reaction is carried out in concentrated aqueous hydrochloric acid solution at a temperature which corresponds to the boiling point of the mixture.

9. Process according to Claim 5, variant b), characterized in that the reaction is carried out in an alcohol having 1-4 carbon atoms, in the presence of alkali metal hydroxide.

10. Process according to Claim 5, variant c), characterized in that a halide of the general formula VIII is used as reactive derivative of a compound of the general formula VI, the meaning of R, Z and X being that indicated in Claim 5.

**Claims for the Contracting States:** AT

1. Process fot the preparation of 2-pyridine-thiol derivatives of the general formula I

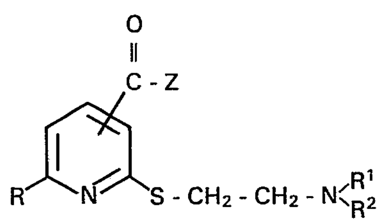

(I)

in which

R represents a hydrogen atom or an alkyl group having 1-4 carbon atoms,

Z represents a phenyl group which is optionally substituted by one or more halogen atoms and/or represents a phenyl group which is substituted by an alkyl group having 1-4 carbon atoms,

$R^1$ and $R^2$ have, independently of one another, the meaning of a hydrogen atom, an alkyl group having 1-4 carbon atoms, an alkyl-phenyl group having 7-11 carbon atoms or a group of the general formula $R-\overset{\overset{\textstyle O}{\|}}{C}-$ ,

the meaning of R being the above, and the substituent $-\overset{\overset{\textstyle O}{\|}}{C}-Z$ is bonded to the 3 or 4 position of the pyridine ring, with the delimitation that if both $R^1$ and $R^2$ have the meaning of a methyl group, and the meaning of Z is a 4-chloro-phenyl group, R does not denote hydrogen atom

and their acid addition salts, characterized in that,

a) a 2-halogeno-pyridine derivative of the general formula II

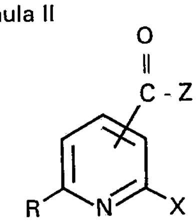

(II)

in which the meaning of R and Z is that indicated in the preamble and X represents a halogen atom

is reacted with a thiol compound of the general formula III

$$HS-CH_2-CH_2-N\langle^{R^1}_{R^2} \qquad (III)$$

in which the meaning of $R^1$ and $R^2$ is that indicated in the preamble

or its acid addition salt; or

b) a pyridine-2-thione derivative of the general formula IV

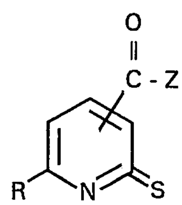

(IV)

in which the meaning of R and Z is that indicated in the preamble

is reacted with a 2-halogeno-ethyl-amine derivative of the general formula V

$$X-CH_2-CH_2-N\langle^{R^1}_{R^2} \qquad (V)$$

in which the meaning of $R^1$ and $R^2$ is that indicated in the preamble, and that of X is halogen atom

or its acid addition salt; or

c) the reactive derivative of a 2-hydroxy-ethyl--thio-pyridine compound of the general formula VI

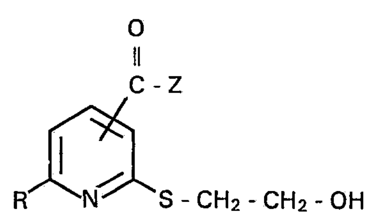

(VI)

in which the meaning of R and Z is that indicated in the preamble

is reacted with an amine of the general formula VII

$$HN\langle^{R^1}_{R^2} \qquad (VII)$$

and which $R^1$ and $R^2$ have the meaning indicated in the preamble; or

d) for the preparation of compounds of the general formula I in which $R^1$ and $R^2$ both represent a hydrogen atom, and Z and R have the meaning indicated in the preamble,

a 2-halogeno-ethyl-thio-pyridine derivative of the general formula VIII

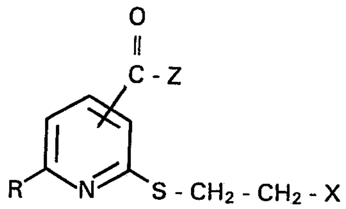

(VIII)

in which the meaning of R and Z is that indicated in the preamble, and that of X is halogen atom

is reacted with potassium phthalimide, and the phthaloyl protective group is removed in a manner known per se, and, if desired the $R^1$ or $R^2$ substituent of a compound of the general formula I prepared in process a), b), c) or d) is converted into another $R^1$ or $R^2$ substituent which falls under a definition given in the preamble, and/or, if desired, a resulting compound of the general formula I is converted into its acid addition salt or is liberated from its acid addition salt.

2. Process according to Claim 1, variant a), characterized in that the reaction is carried out in a solvent in the presence of an acid-binding agent.

3. Process according to Claim 2, characterized in that an alcohol having 1-4 carbon atoms is used as solvent, and alkali metal hydroxide is used as acid-binding agent.

4. Process according to Claim 1, variant a), characterized in that the reaction is carried out in concentrated aqueous hydrochloric acid solution at a temperature which corresponds to the boiling point of the mixture.

5. Process according to Claim 1, variant b), characterized in that the reaction is carried out in an alcohol having 1-4 carbon atoms, in the presence of alkali metal hydroxide.

6. Process according to Claim 1, variant c), characterized in that a halide of the general formula VIII is used as reactive derivative of a compound of the general formula VI, the meaning of R, Z and X being that indicated in Claim 5 (sic).

7. Process for the preparation of a pharmaceutical composition, characterized in that the compounds prepared according to Claims 1 to 6, or their pharmaceutically acceptable addition salts, are formulated as active ingredients with appropriate excipients.

**Revendications pour les Etats contractants:**
BE, CH, DE, FR, GB, IT, LI, NL, SE

1. Dérivés du pyridinethiol-2 répondant à la formule générale I

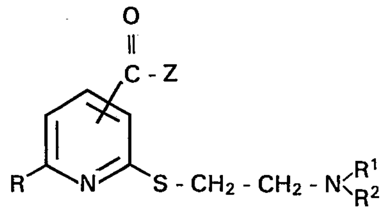

(I)

dans laquelle

R représente un atome d'hydrogène ou un groupe alkyle comportant 1 à 4 atomes de carbone,

Z représente un groupe phényle éventuellement substitué par un ou plusieurs atomes d'halogène et/ou un groupe phényle substitué par un groupe alkyle comportant 1 à 4 atomes de carbone,

$R^1$ et $R^2$ représentent, indépendamment l'un de l'autre, un atome d'hydrogène, un groupe alkyle comportant 1 à 4 atomes de carbone, un groupe alkylphényle comportant 7 à 11 atomes de carbone ou un groupe répondant à la formule générale R-C- ,
||
O

R ayant la signification précitée, et le substituant -C-Z est fixé en position 3 ou 4 au noyau pyridinique,
||
O

avec la condition que, si $R^1$ et $R^2$ représentent tous deux un groupe méthyle et si Z représente un groupe 4-chloro-phényle, R ne représente pas un atome d'hydrogène,

et leurs sels d'addition d'acides.

2. 2-([2-Aminoéthyl]-thio)-3-benzoyl-pyridine, et son chlorhydrate.

3. 3-Benzoyl-2-{(2-[N,N-diacéthylamino]-éthyl)--thio}-pyridine.

4. Produit pharmaceutique, caractérisé en ce qu'il renferme, comme agent actif, au moins un composé répondant à la formule générale I, où R, Z, $R^1$ et $R^2$ ont la signification indiquée dans la revendication 1, ou son sel d'addition d'acides thérapeutiquement acceptable, en plus de supports usuels.

5. Procédé de préparation de dérivés du pyridine--thio-2 répondant à la formule générale I

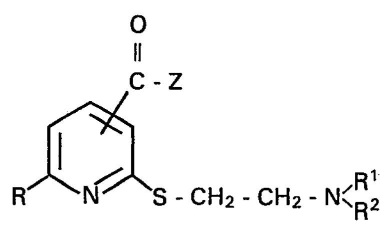

(I)

dans laquelle

R représente un atome d'hydrogène ou un groupe alkyle comportant 1 à 4 atomes de carbone,

Z représente un groupe phényle éventuellement substitué par un ou plusieurs atomes d'halogène et/ou un groupe phényle substitué par un groupe alkyle comportant 1 à 4 atomes de carbone,

$R^1$ et $R^2$ représentent, indépendamment l'un de l'autre, un atome d'hydrogène, un groupe alkyle comportant 1 à 4 atomes de carbone, un groupe alkyl-phényle comportant 7 à 11 atomes de carbone ou un groupe répondant à la formule générale R-C- ,
||
O

R ayant la signification précitée, et le substituant $-\overset{\overset{O}{\|}}{C}-Z$ est fixé en position 3 ou 4 au noyau pyridinique, avec pour condition que, si $R^1$ et $R^2$ représentent tous deux un groupe méthyle et si Z représente un groupe 4-chloro-phényle, R ne représente pas un atome d'hydrogène,

et leurs sels d'addition d'acides, caractérisé en ce que

a) on fait réagir un dérivé 2-halopyridinique répondant à la formule générale II

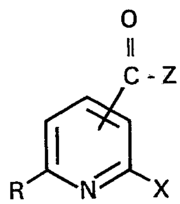

(II)

dans laquelle la signification de R et Z est indiquée dans le préambule et X représente un atome d'halogène,

sur un composé thiolique répondant à la formule générale III

$$HS-CH_2-CH_2-N{<}^{R^1}_{R^2} \qquad (III)$$

dans laquelle $R^1$ et $R^2$ ont la signification indiquée dans le préambule, ou sur son sel d'addition d'acide; ou bien

b) on fait réagir un dérivé pyridine-2-thionique répondant à la formule générale IV

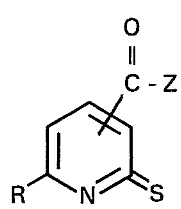

(IV)

dans laquelle R et Z ont la signification indiquée dans le préambule, sur un dérivé 2-haloéthylaminique répondant à la formule générale V

$$X-CH_2-CH_2-N{<}^{R^1}_{R^2} \qquad (V)$$

$R^1$ et $R^2$ ayant la signification indiquée dans le préambule, X représentant un atome d'halogène,

ou sur son sel d'addition d'acide; ou bien

c) on fait réagir le dérivé réactif d'un composé 2-hydroxy-éthyl-thio-pyridinique répondant à la formule générale VI

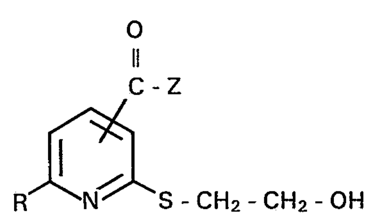

(VI)

dans laquelle R et Z ont la signification indiquée dans le préambule, sur une amine répondant à la formule générale VII

$$HN{<}^{R^1}_{R^2} \qquad (VII)$$

dans laquelle $R^1$ et $R^2$ ont la signification indiquée dans le préambule;

d) pour préparer des composés répondant à la formule générale I, dans laquelle $R^1$ et $R^2$ représentent tous deux un atome d'hydrogène, Z et R ont la signification indiquée dans le préambule,

on fait réagir un dérivé 2-haloéthylthiopyridinique répondant à la formule générale VIII

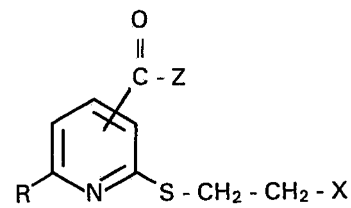

(VIII)

dans laquelle R et Z ont la signification indiquée dans le préambule,

X représente un atome d'halogène,

sur du phtalimide-potassé, puis l'on élimine de façon connue en soi le groupe phtaloyle protecteur, et

on convertit, si on le souhaite, le substituant $R^1$ ou $R^2$ d'un composé répondant à la formule générale I, préparé par le procédé a), b), c) ou d), en un autre substituant $R^1$ ou $R^2$ qui entre dans le cadre d'une définition donnée dans le préambule, et/ou

on convertit, si on le souhaite, un composé obtenu, répondant à la formule générale I, en son sel d'addition d'acide ou bien on le libère de son sel d'addition d'acide.

6. Procédé selon la revendication 5, variante a), caractérisé en ce qu'on effectue la réaction dans un solvant, en présence d'un fixateur d'acide.

7. Procédé selon la revendication 6, caractérisé en ce qu'on utilise, comme solvant, un alcool comportant 1 à 4 atomes de carbone et, comme fixateur d'acide, un hydroxyde de métal alcalin.

8. Procédé selon la revendication 5, variante a), caractérisé en ce qu'on effectue la réaction en solution chlorhydrique aqueuse concentrée, à une température correspondant au point d'ébullition du mélange.

9. Procédé selon la revendication 5, variante b), caractérisé en ce qu'on effectue la réaction dans un alcool à 1 à 4 atomes de carbone, en présence d'un hydroxyde de métal alcalin.

10. Procédé selon la revendication 5, variante c), caractérisé en ce qu'on utilise, comme dérivé réactif d'un composé répondant à la formule générale VI, un halogénure répondant à la formule générale VIII, R, Z et X ayant la signification indiquée dans la revendication 5.

**Revendications pout l'Etat contractant:** AT

1. Procédé de préparation de dérivés du pyridine-thiol-2 répondant à la formule générale I

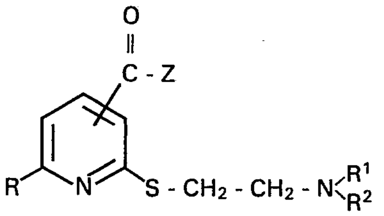

(I)

dans laquelle

R représente un atome d'hydrogène ou un groupe alkyle comportant 1 à 4 atomes de carbone,

Z représente un groupe phényle éventuellement substitué par un ou plusieurs atomes d'halogène et/ou un groupe phényle substitué par un groupe alkyle comportant 1 à 4 atomes de carbone,

$R^1$ et $R^2$ représentent, indépendamment l'un de l'autre, un atome d'hydrogène, un groupe alkyle comportant 1 à 4 atomes de carbone, un groupe alkylphényle comportant 7 à 11 atomes de carbone ou un groupe répondant à la formule générale $R-\underset{\underset{O}{\|}}{C}-$,

R ayant la signification précitée, et le substituant $-\underset{\underset{O}{\|}}{C}-Z$ est fixé en position 3 ou 4 au noyau pyridinique,

avec pour condition que, si $R^1$ et $R^2$ représentent tous deux un groupe méthyle et si Z représente un groupe 4-chloro-phényle, R ne représente pas un atome d'hydrogène,

et leurs sels d'addition d'acides, caractérisé en ce que

a) on fait réagir un dérivé 2-halopyridinique répondant à la formule générale II

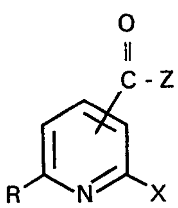

(II)

dans laquelle la signification de R et Z est indiquée dans le préambule et X représente un atome d'halogène,

sur un composé thiolique répondant à la formule générale III

$$HS-CH_2-CH_2-N{<}^{R^1}_{R^2} \qquad (III)$$

dans laquelle la signification de $R^1$ et $R^2$ est indiquée dans le préambule, ou sur son sel d'addition d'acide; ou bien

b) on fait réagir un dérivé pyridine-2-thionique répondant à la formule générale IV

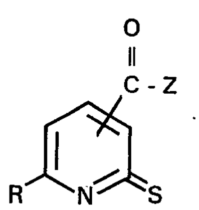

(IV)

dans laquelle R et Z ont la signification indiquée dans le préambule, sur un dérivé 2-haloéthylaminique répondant à la formule générale V

$$X-CH_2-CH_2-N{<}^{R^1}_{R^2} \qquad (V)$$

$R^1$ et $R^2$ ayant la signification indiquée dans le préambule X représentant un atome d'halogène,

ou sur son sel d'addition d'acide; ou bien

c) on fait réagir le dérivé réactif d'un composé 2-hydroxy-éthyl-thio-pyridinique répondant à la formule générale VI

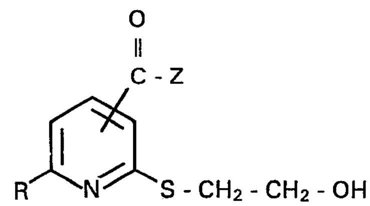

(VI)

dans laquelle R et Z ont la signification indiquée dans le préambule, sur une amine répondant à la formule générale VII

$$HN{<}^{R^1}_{R^2} \qquad (VII)$$

dans laquelle $R^1$ et $R^2$ ont la signification indiquée dans le préambule;

d) pour préparer des composés répondant à la formule générale I, dans laquelle $R^1$ et $R^2$ représentent tous deux un atome d'hydrogène, Z et R ont la signification indiquée dans le préambule,

on fait réagir un dérivé 2-haloéthylthiopyridinique répondant à la formule générale VIII

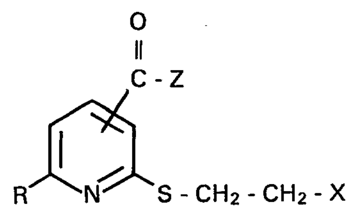

(VIII)

dans laquelle R et Z ont la signification indiquée dans le préambule,

X représente un atome d'halogène,

sur du phtalimide-potassé, puis l'on élimine de façon connue en soi le groupe phtaloyle protecteur, et

si on le souhaite, on convertit, le substituant $R^1$ ou $R^2$ d'un composé répondant à la formule générale I, préparé par le procédé a), b), c) ou d), en un autre substituant $R^1$ ou $R^2$ qui entre dans le cadre d'une définition donnée dans le préambule, et/ou

si on le souhaite, on convertit un composé obtenu, répondant à la formule générale I, en son sel d'addition d'acide ou bien on le libère de son sel d'addition d'acide.

2. Procédé selon la revendication 1, variante a), caractérisé en ce qu'on effectue la réaction dans un solvant, en présence d'un fixateur d'acides.

3. Procédé selon la revendication 2, caractérisé en ce qu'on utilise, comme solvant, un alcool comportant 1 à 4 atomes de carbone et, comme fixateur d'acide un hydroxyde de métal alcalin.

4. Procédé selon la revendication 1, variante a), caractérisé en ce qu'on effectue la réaction en solution chlorhydrique aqueuse concentrée à une température correspondant au point d'ébullition du mélange.

5. Procédé selon la revendication 1, variante b), caractérisé en ce qu'on effectue la réaction dans un alcool à 1 à 4 atomes de carbone, en présence d'un hydroxyde de métal alcalin.

6. Procédé selon la revendication 1, variante c), caractérisé en ce qu'on utilise, comme dérivé réactif d'un composé répondant à la formule générale VI, un halogénure répondant à la formule générale VIII, R, Z et X ayant la signification indiquée dans la revendication 5.

7. Procédé de préparation d'un produit pharmaceutique, caractérisé en ce qu'on formule les composés préparés selon les revendications 1 à 6 ou leurs sels d'addition d'acides pharmaceutiquement acceptables sous la forme d'agents actifs avec des excipients correspondants.